# EUROPEAN PATENT SPECIFICATION

(11) **EP 2 666 503 B1**
(45) Date of publication and mention of the grant of the patent: **29.06.2016**
(21) Application number: 13169269.1
(22) Date of filing: 24.05.2013
(51) Int. Cl.: A61M 15/00

(54) **An inhaler mechanism having a transmission and a pivot arm**
Inhalator mit einer Übertragung und einem Schwenkarm
Mécanisme d'inhalateur comprenant une transmission et un bras de pivot

(30) Priority: 25.05.2012 TR 201206167; 08.02.2013 TR 201301562; 15.02.2013 TR 201301847; 19.02.2013 TR 201301950; 26.03.2013 TR 201303661; 29.04.2013 TR 201305053; 09.05.2013 TR 201305562
(43) Date of publication of application: 27.11.2013
(73) Proprietor: Arven Ilac Sanayi Ve Ticaret A.S., Istanbul 34460 (TR)
(72) Inventor: Toksöz, Zafer, 34460 Istanbul (TR); Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(56) References cited:
- WO-A1-2010/114504
- WO-A1-2010/114506
- US-A1- 2004 050 385
- US-A1- 2007 215 149
- US-A1- 2009 283 095

## Description

### Field of Invention

The present invention relates to a device for administering dry powder inhalation drugs.

The present invention particularly relates to improvements made in the blister advancement mechanism of dry powder inhaler devices.

### Prior Art

Diseases such as asthma, bronchitis, and COLD (Chronic Obstructive Lung Disease) substantially decrease the quality of human life, despite the developments which have been carried out in the diagnosis and therapy thereof in the recent years. It has been proposed to administer medicaments via inhalers for optimizing the treatment of such diseases. The inhaler route of treatment is the most preferred one and it is expected to remain so, as the first option, in the future. The most important advantage of using medicaments via inhalation is based on providing a more efficient therapy by making use of lesser medicaments, delivering higher concentrations of medicaments to the airways, and particularly decreasing the systemic side effects of medicaments. The most important causes of the lack of a satisfactory control of patients albeit the presence of quite efficient treatments against respiratory tract diseases are stated to be as the noncompliance, arising from the inefficient use of inhalers and from inadequate compliance to the physician-recommended treatments.

There have been developed various inhaler devices for administering inhalation drugs nowadays. These devices are basically classified into two groups, i.e. metered dose inhalers and dry powder inhalers. Such type of devices are structurally provided with such basic components as an actuator, counter, housing, lid, lock, etc.. Additionally, powder inhalation drugs are kept in reservoirs or containers such as blisters, capsules, etc.. Blisters are structured from two basic parts, a main layer provided with cavities holding the drug, and a strippable protective layer.

Such devices comprise an outer body and a lid provided thereon, an inner body disposed in the outer body, a blister strip placed in the inner body, a lower holder or reservoir receiving the strip in a rolled form, as well as gears and a gear set connected to each other in a functionally compatible manner to actuate the blister strip so that the main layer thereof comprising the cavities is wound and stored after it is separated into its layers. Push member or trigger mechanism have been developed to actuate this mechanism.

In inhaler devices with a plurality of blisters, a force is exerted to the mechanism actuating the blister by means of the trigger or the push member such that the mechanism is actuated. In practice, however, various problems are encountered in the mechanisms. Some of these are encountered in the triggers actuating the mechanism. These triggers are disadvantageous in terms of use difficulty, and require an extra movable volume on the exterior of the inhaler device. The linear motion of trigger mechanisms, which are advantageous in terms of volume and are slid into the interior of the device, is converted into a rotational motion by means of a gear to which it is connected. In the applications WO2010114506, WO2010114505, for instance, the linear motion performed by a trigger is converted into a rotational motion by means of a wheel. This trigger is slid into a slot in the body of the inhaler device to perform axial motion, and the force generated by this motion is transferred to a blister advancement mechanism by means of a gear with which it meshes. However, the gears providing the transmission and conversion of this motion are exposed to overloads. Additionally, losses are encountered in force transmission when (an) extra gear(s) is/are used. The elimination of this loss, in turn, requires the exertion of an extra amount of force. Another drawback is that slackness occurs between the gears and the contact surfaces. This slackness and extra force exertion, in turn, result in a greater problem in that the gears and wheels in the mechanism become worn out or even break.

The use of the currently available inhaler devices necessitates certain training and practice. The development of inhaler devices always occurs in the form of practical systems, providing the patients with improved convenience of use. Selecting the proper device for a respective patient is also an important issue. Many criteria, such as a patient's cognitive and physical efficiency, ease of use, safety and price, etc. are considered while a device is selected.

US 2009/0283095 A1 discloses a fluid-product dispensing device. The dispensing device comprises: a body; at least one cover element that is movable between a closed position and an open position; individual reservoirs formed on a reservoir substrate; movable support means that receive said reservoir substrate and that are displaceable between a non-dispensing position and a dispensing position; and an indicator device for indicating doses that have been dispensed or that remain to be dispensed, said indicator device comprising: a rotary indicator element that supports indicator means, such as numbers, colors, and/or symbols; and an actuator that co-operates with said movable support means so as to cause said rotary indicator element to turn when said movable support means return from the dispensing position to the non-dispensing position.

In result, there is a novelty required in the field of inhaler devices, providing high-accuracy operation, advantages in terms of cost, volume, and use, as well as minimizing the failures and damages which can occur in the device.

### Objects and Brief Description of Invention

The present invention relates to an improved inhaler device for use with dry powder inhaling purposes, eliminating all aforesaid problems and bringing additional advantages to the relevant prior art.

Accordingly, the main object of the present invention is to provide a dry powder inhaler device, which can be operated at a desired accuracy, can peel off a blister for use, and can perform this process faultlessly as compared to similar devices.

Another object of the present invention is to reduce the amount of force to be exerted to the trigger of the device to operate the same.

Another object of the present invention is to prevent the wearing or breaking of the components in the mechanism as a result of reducing the amount of force exerted to the trigger and thus to the gears and the gear mechanism.

In order to achieve the objects referred to above and to emerge in the following detailed description, a dry powder inhaler device is developed which comprises a body and a trigger moving on an linear direction in the body, a main drum into which a blister strip having drug-carrying cavities is placed, a gear set which enables to release a drug in the next cavity of the blister strip to be administered with rotating the main drum and around which a protective layer of the blister strip is wound, and additional gears which are in connection with the main drum and around which a main layer of the blister strip is wound.

The invention is defined in the appended claim 1.

According to the present invention a pivot arm is connected to the body by means of a support/pin between a first end and a second end of the pivot arm so that the pivot arm can only rotate around the support pin and which converts the axial motion received from the trigger into a rotational motion on its second end by means of a pin disposed on the trigger, and a transmission arm which has a first end connected to the drum in a rotating manner and a second end comprising a pin disposed so as to rest against the second end of the pivot arm and which transmits the rotational motion received from the pivot arm to the drum in a reversed direction for converting the axial/linear motion of the trigger into rotational motion and transfer this motion to the gear set.

A preferred embodiment according to the present invention comprises a barrier restricting the rotation of the pivot arm.

In a preferred embodiment according to the present invention, the second end of the pivot arm has a triangular shape.

In a further preferred embodiment according to the present invention, the pin has a semicircular profile.

In a preferred embodiment according to the present invention, the main drum is coupled to a transmission wheel disposed on the transmission arm.

A preferred embodiment according to the present invention comprises a pretensioned spring which restores the trigger to its initial position after it is pushed in axially and released by a user.

In a preferred embodiment of the present invention, the spring is disposed at a lower side of the transmission wheel.

In a preferred embodiment according to the present invention, the pin provided on the second end of the transmission arm is moved by sliding on the surface of the second end of the pivot arm having a triangular shape.

In a preferred embodiment of the present invention, the pivot arm and the transmission arm are disposed between the main drum and the trigger.

Structural and characteristic features, and all advantages of the present invention shall be made clear by means of annexed figures described here below and a detailed description written by making references to said figures; therefore, the present invention must be evaluated by taking into consideration these figures and the detailed description as well.

### Brief Description of Figures

Figure 1 is an illustration of an exemplary embodiment according to the present invention.
Figure 2 is a representative embodiment of an outer body and an inner body according to the present invention.
Figure 3 is a representative embodiment of the outer body, inner body, and a mechanism according to the present invention.
Figure 4 is a representative embodiment of the outer body and an the mechanism according to the present invention.
Figure 5 is a representative embodiment of the outer body and an the mechanism according to the present invention.
Figure 6 is a representative embodiment of the mechanism according to the present invention.
Figure 7 is a representative embodiment of the mechanism according to the present invention.
Figure 8 is a representative embodiment of the inner body according to the present invention.
Figure 9a is a representative embodiment of the main drum according to the present invention.
Figure 9b is a representative embodiment of the transmission wheel and the transmission arm according to the present invention.

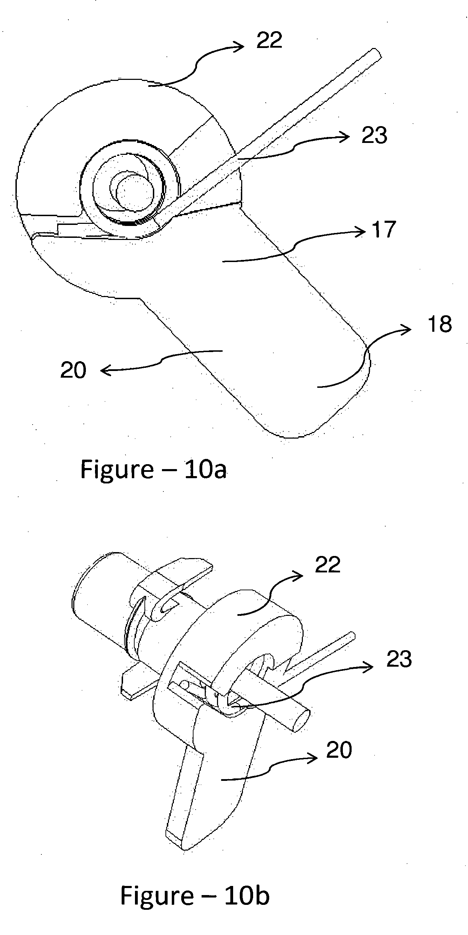

Figure 10a is a representative embodiment of the transmission wheel, the transmission arm and the spring according to the present invention.
Figure 10b is a representative embodiment of the transmission wheel, the transmission arm and the spring according to the present invention.
Figure 11 a is a representative embodiment of blister cover winding gears according to the present invention.

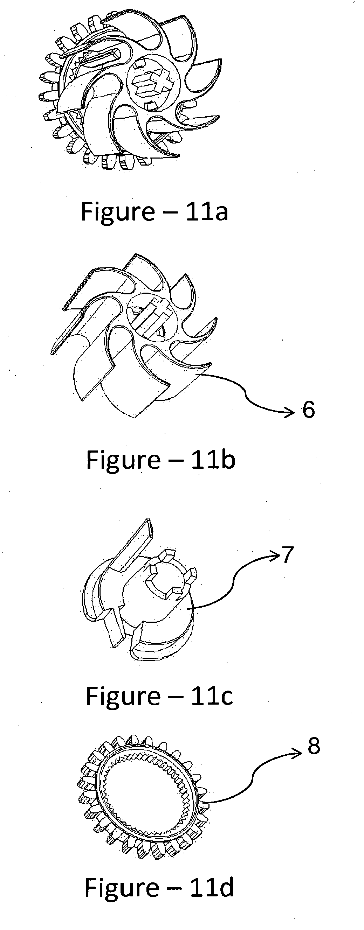

Figure 11 b is a representative embodiment of a blister cover upper winding gear according to the present invention.
Figure 11 c is a representative embodiment of a blister cover central winding gear according to the present invention.
Figure 11 d is a representative embodiment of a blister cover lower winding gear according to the present invention.

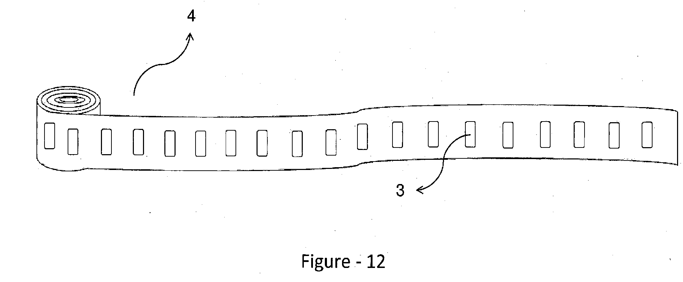

Figure 12 is a representative embodiment of a blister according to the present invention.

### Reference Numbers in Figures

- 1.: Outer body
- 2.: Trigger
- 3.: Cavity
- 4.: Blister
- 5.: Main drum
- 6.: Blister cover upper winding gear
- 7.: Blister cover central winding gear
- 8.: Blister cover lower winding gear
- 9.: First additional gear
- 10.: Second additional gear
- 11.: Inhaler device
- 12.: First end of the pivot arm
- 13.: Second end of the pivot arm
- 14.: Support pin
- 15.: Trigger pin
- 16.: Pivot arm
- 17.: First end of the transmission arm
- 18.: Second end of the transmission arm
- 19.: Pin of the transmission arm
- 20.: Transmission arm
- 21.: Barrier
- 22.: Transmission wheel
- 25.: Inner body
- 26.: Lower reservoir
- 27.: Left medial reservoir
- 28.: Right medial reservoir
- 29.: Upper reservoir
- 30.: Trigger channel

### Detailed Description of Invention

In the following detailed description, an inhaler device (11) according to the present invention shall be described illustratively by making references to annexed figures, only to make it clear without imposing any restrictions thereon.

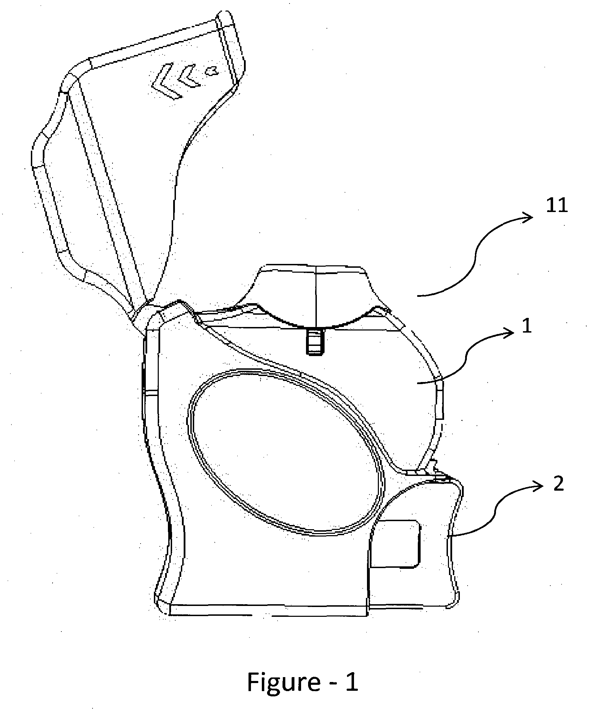

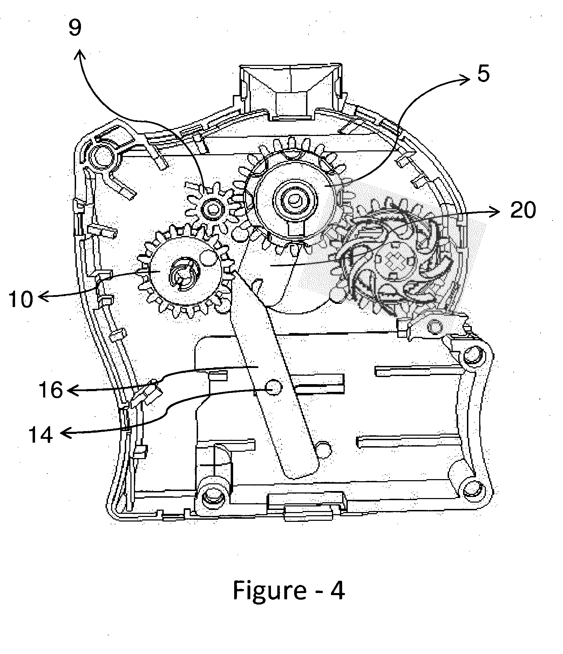

An outer body (1) of the inhaler device (11) according to the present invention as illustrated in figures 1, 2, 4, 9a, 9b, 11 a, 11 b, 11c, and 11 d is obtained by assembling two compatible parts together. The interior of said parts comprises both fastening tabs to fasten them together, and reservoirs and pins, allowing the placement of the mutually connected main drum and transmission wheel (5, 22) and the mutually connected blister cover winding gear set (6, 7, 8) in the outer body (1). The upper part of the body is provided with a mouthpiece having a drug outlet opening. The lower part of the body is provided with a trigger (2) slid into the body.

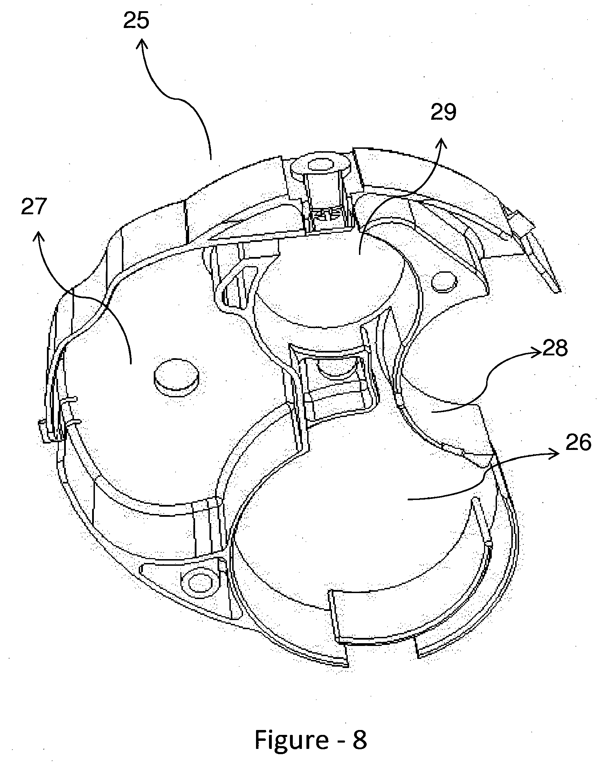

An inner body (25) is positioned in the interior of the outer body (1) to place a blister (4) therein, as illustrated in figures 8 and 12. This single-piece inner body (25) comprises lower, left and right medial and upper reservoirs (26, 27, 28, 29), respectively, along with recessed surfaces and fastening tabs. An unused blister (4) is stored in the lower reservoir (26), this strip-shaped blister (4) being extended along intermediate channels and subsequently separated into two parts, i.e. a main layer and a protective layer, by means of the mechanism. The main layer with one end fastened to a second additional gear (10) is stored in the left medial reservoir (27) where this second additional gear (10) is positioned. The end of the protective layer, in turn, is fastened to a pin provided on the central winding gear (7).

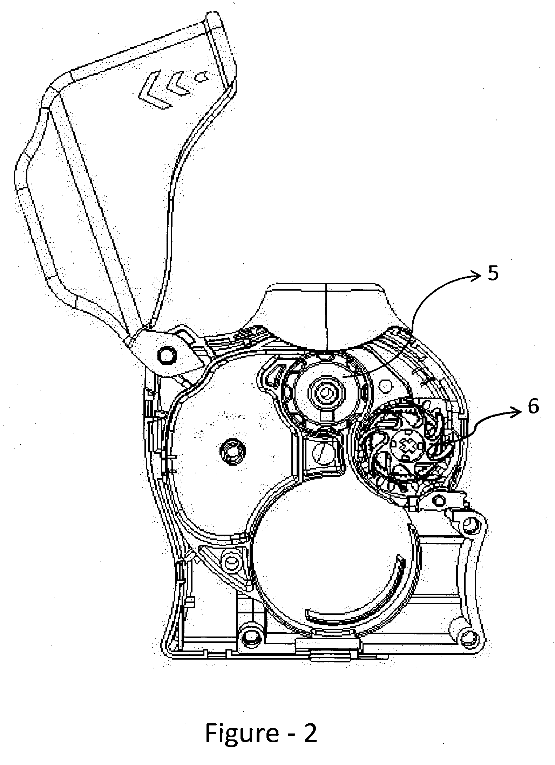

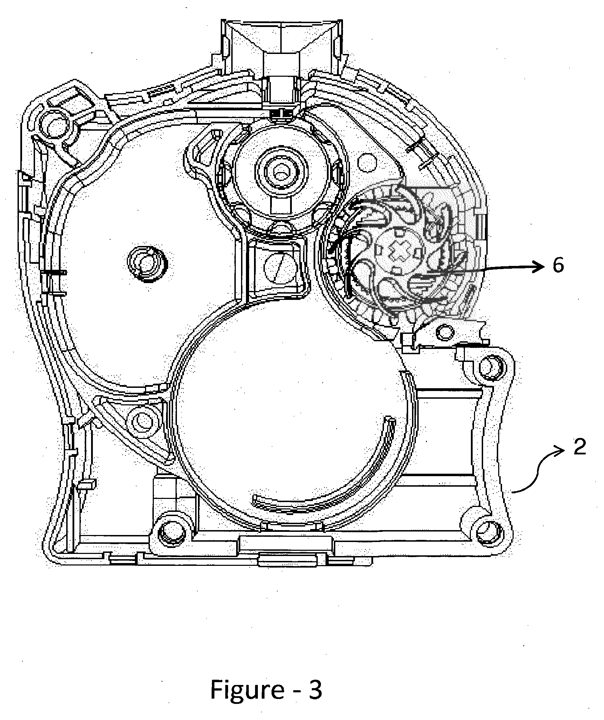

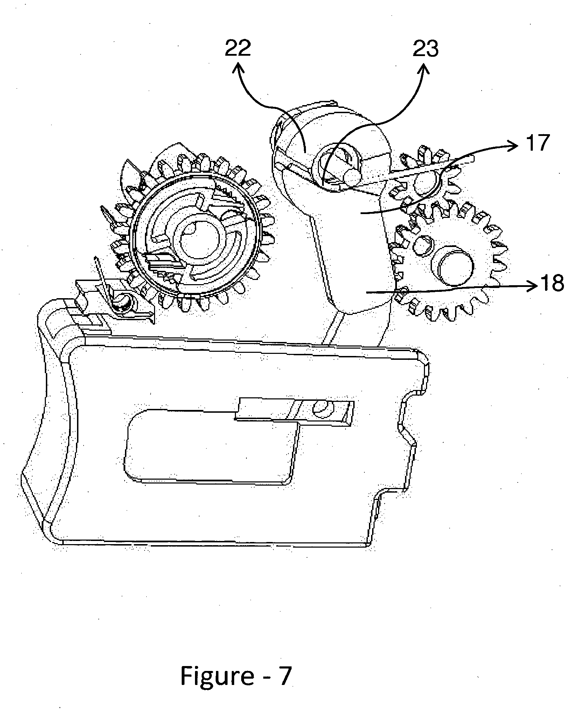

As illustrated in figures 2, 3 and 7, at the beginning of the blister (4) advancement mechanism is provided a trigger (2), slid into a lower side of said outer body (1). The trigger (2) is capable to perform an axial/linear motion in the inner part of the body (1). A grip surface provided on the exterior of the trigger (2), which is the surface by which a user exerts force to push in said trigger, is formed in an incurved manner to provide ease of use. A spring is placed into a spring holder formed at a lower side of the transmission wheel (22), this spring becoming compressed (i.e. loaded) when the trigger (2) is pushed into the body (1). The spring has a helical form. One end of this helical spring rests against a wall of the spring holder formed on a lower side of the transmission wheel and the other end thereof leans against an inner surface of the body (1).

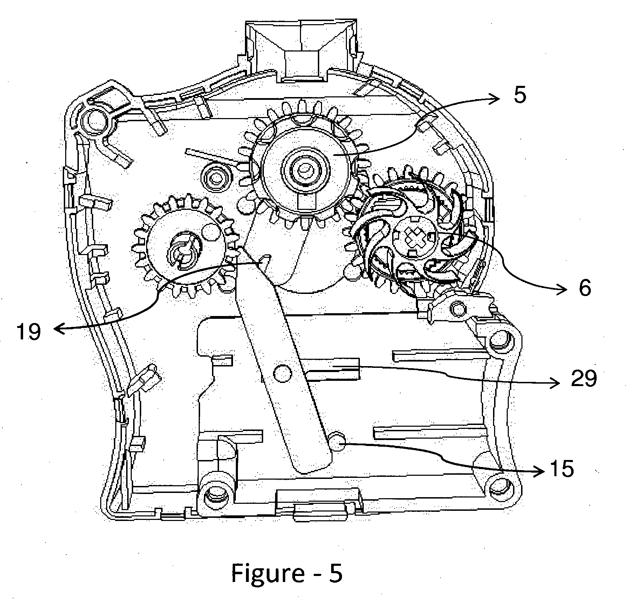

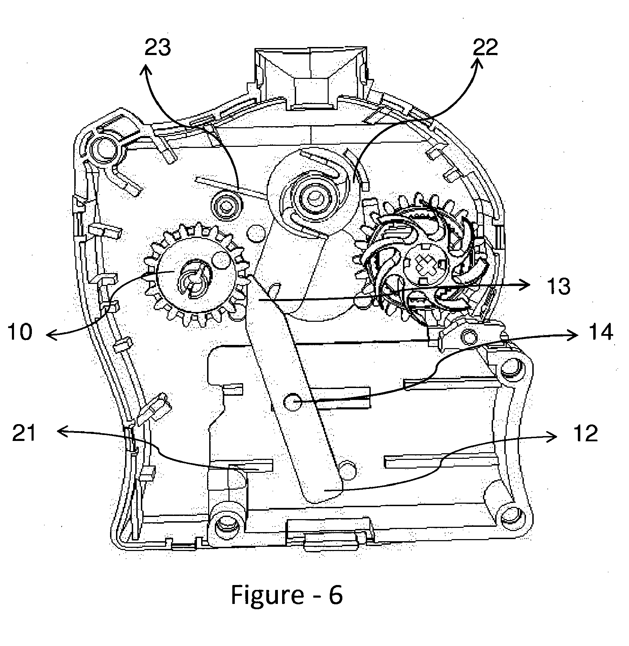

As illustrated in figures 5, 6, 7 10a and 10b a rectangular cutout (30) is formed on the trigger. A support pin (14) fixed to the inner part of the body (1) is passed through this cutout. The central part or a point close to the center of a pivot arm (16) is connected to the support pin (14) which passes through the cutout (30) in the body of the trigger (2). This arm (16) is connected to the support pin (14) so as to pivot around the pin (14). A transmission arm (20) is connected to the pivot arm (16). This connection is provided by means of a pin (19) disposed on the second end (18) of the transmission arm. The second end (13) of the pivot arm is shaped exactly like a triangle. The pin (19) disposed at the tip of the transmission arm can move by sliding forth and back on the surface of the triangular tip having beveled edges. Thus, the pivot arm (16) and the transmission arm (20) which are in contact are moved upward and downward, thereby performing a folding and unfolding movement. In other words, the pivot arm and the transmission arm are moved like a scissor to actuate the mechanism. A transmission wheel (22) is provided on the tip part of the transmission arm (20). This transmission wheel (22) is engaged to a main drum (5). The main drum (5) is mounted over the transmission wheel (22) so as to move in the same way with the wheel.

According to the details given above, the operation of the device according to the present invention is as follows. Following the opening of the lid of the device, a force is exerted by the user to the grip surface of the trigger. Then the trigger (2) is slid into the interior of the body (1). The pivot arm (16) is actuated with the trigger (2) moving axially/linearly and the pin (15) connected to the trigger pushing the pivot arm. The pivot arm (16) rotates around the support pin (14) which is passed through an opening on the middle part of the pivot arm. This is not a complete rotational motion. The arm performs a rotational motion at an angle lower than 360 degrees. The limit of the movement is defined by the pin (15) on the trigger and a barrier (21) provided on the trigger. This motion of the pivot arm is transmitted to the transmission arm (20) which is coupled to the pivot arm (16). Under the influence of the pivot arm (16), the transmission arm (20) performs a rotational motion through the transmission wheel (22) with which it is coupled. With this motion of the transmission arm (20), the transmission wheel (22) provided on one end thereof is actuated. With the movement of the transmission wheel (22), the main drum (5) disposed over the wheel is rotated so as to rotate the gears with which it meshes. Thus, a blister placed around the gears is moved and the cavities (3) of the blister are opened.

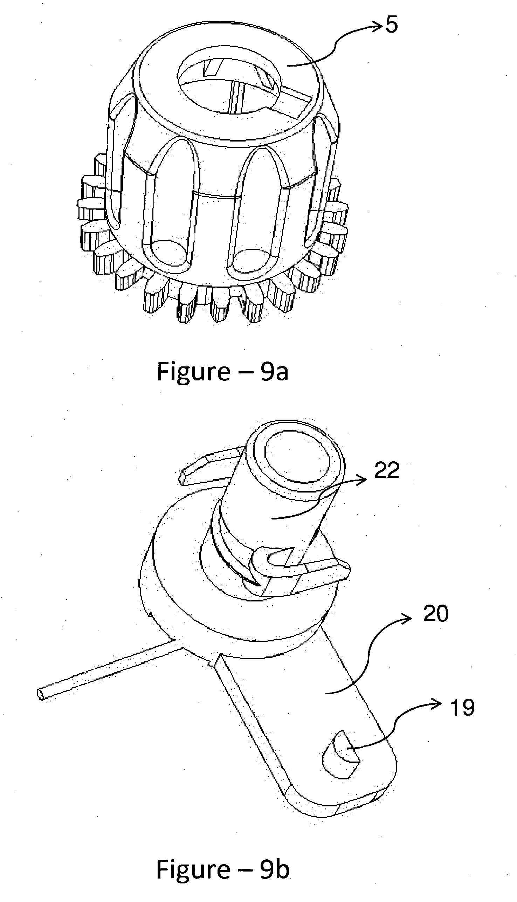

As illustrated in detail in Figure 9a, the main drum (5) moves on a single direction and provides only the advancement of the blister (4). The transmission wheel (22) below the main drum is capable to rotate forward and backward.

The transmission wheel (22) transmits this motion to the main drum (5) and to the first and second additional gears (9, 10). While the transmission wheel (22) and the main drum (5) rotate counterclockwise, the blister cover lower winding gear (8) and the blister cover central and upper winding gears (7, 6) disposed above the lower winding gear rotate clockwise. The blister (4) in contact with these gears is advanced in the channels of the inner body (25), is passed through and stripped or peeled off between the gear sets so that the drug in the respective cavity is released. The main layer of the blister, now separated into two parts, is rolled in the left medial reservoir (27), whereas the protective or cover layer of the blister is wound around the blister cover upper gear (6).

As a result of sliding the trigger (2) into the interior of the outer body (1), a retaining groove provided on the trigger (2) is coupled and fastened to a locking clips lug provided just over the groove, resulting in the administration of a single dose of medicament. Keeping this slide-in action until the locking position is achieved ensures a complete peeling-off of the blister and an accurate administration of the required dosage amount. As a result of this locking effect, the trigger (2) becomes retained and it remains out-of-use for a short period of time. This slide-in action also causes one end of the spring to become compressed at the lower surface of the transmission wheel (22) by resting against the inner surface of the body (1).

After the user inhales the powder drug, he/she closes the lid of the device, so that a tip part of the lid exerts force to a rear part of the locking clips, the tip thereof is lifted above, and the clips lug and the retaining groove are detached from each other. As a result of this, the compressed spring rotates the transmission wheel (22) backward and makes the mechanism move backward too. Thus, the device is restored for the next use without requiring any user intervention. However, the mechanism does not move the blister backward. It can be seen from the disclosure above, that the device according to the present invention can be operated by a simple single push action of the user. When the lid is closed, the device (11) is set up automatically and is restored for the next use.

The linear motion of the trigger is the sliding in-out motion of the trigger on any direction, including those on the x and y axes. This direction is determined according to the design of the device.

The term gear set covers the main drum, transmission wheel, blister cover lower, central, and upper winding gears, and the additional gears, wherein other wheels and gears to be included in the basic elements of the device are also covered under this term.

| | **F₀ (N)** | **F_{B} (N)** | **F₁ (N)** | **F₁₅ (N)** | **I (mm)** | **M_{b} (Nmm)** | **f_{B} (N)** | **M₁ (Nmm)** | **f₁ (N)** | **M₁₅ (Nmm)** | **f₁₅ (N)** |
|---|---|---|---|---|---|---|---|---|---|---|---|
| Device according to the present invention | 8,9 | 16 | 15 | 17 | 19 | 62 | 3,2 | 200 | 10,5 | 300 | 15,7 |
| Device according to the prior art | 11 | 21 | 43 | 60 | 6,2 | 62 | 10 | 200 | 32 | 300 | 49 |

- F0:: Trigger force of a device with the mechanism removed
- FB:: Trigger force of an empty device with a complete mechanism, but without a blister
- F1:: First trigger force of a device loaded with a blister
- F15:: Fifteenth trigger force of a device loaded with a blister without the device being subjected to cleaning
- Mb.....M15:: Force required to rotate the main drum under respective conditions
- fb.......f15:: Force exerted to the main actuating lever or gear to rotate the main drum under respective conditions
- I:: The distance or the arm length which is perpendicular to the rotational axis.
- N :: Newton
- Nmm :: Newton millimeter

As can be seen in the table, the force which is required to be exerted by a user on the trigger of the device according to the present invention to operate the same is reduced almost to two thirds of those of the devices according to the prior art.

In result, an inhaler device is obtained with the embodiment disclosed above, this device being extremely accurate, reliably-operating, and providing advantages in terms of cost and volume, wherein the force required to operate the device and exerted to the components of the mechanism thereof is reduced. Two arms (16, 20) are used in place of a gear between the trigger (2) and the main drum (5) to provide these advantages. The torque generated by means of said arms provides a substantial advantage in the push force required to slide in the trigger.

The design of components used may be varied in alternative embodiments according to the type of device being produced. In result, the protection scope of the present invention is set forth in appended claims and cannot be restricted to the illustrative disclosures given above, under the detailed description. It is obvious that a person skilled in the relevant art can produce similar embodiments under the light of the foregoing disclosures, without departing from the main principles of the present invention.

## Claims

1. A dry powder inhaler device (11) comprising a body (1) and a trigger (2) moving on an linear direction in the body (1), an inner body (25) positioned in the interior of the outer body (1), a main drum (5) into which a blister (4) strip having drug-carrying cavities (3) are placed, a gear set (6, 7, 8) which enables to release drugs contained in the cavities of said blister strip to be administered by rotating the main drum (5) and around which a protective layer or cover of the blister (4) strip is wound, and additional gears (9, 10) which are in connection with the main drum (5) and around which a main layer of the blister (4) strip is wound, **characterized in that** said device further comprises;
- a pivot arm (16) which is connected to the body (1) by means of a support/pin (14) between a first end (12) and a second end (13) of the pivot arm (16) so that the pivot arm (16) is made rotatable around said support pin (14), wherein said pivot arm (16) converts the linear motion from the trigger (2) into rotational motion on its second end (13) by means of a pin (15) disposed on the trigger (2), and
- a transmission arm (20) which has a first end (17) connected to the drum (5) in a rotating manner and a second end (18) comprising a pin (19) disposed so as to rest against the second end (13) of the pivot arm (16), wherein said transmission arm (20) transmits the rotational motion received from the pivot arm (16) to the drum (5)
such that the linear motion of the trigger (2) converted into a rotational motion is transferred to the gear set (6, 7, 8).

2. The dry powder inhaler device (11) according to Claim 1, **characterized in that** the trigger (2) comprises a barrier (21) which restricts the rotation of the pivot arm (16).

3. The dry powder inhaler device (11) according to any of the preceding claims, **characterized in that** the second end (13) of the pivot arm (16) has a triangular shape.

4. The dry powder inhaler device (11) according to any of the preceding claims, **characterized in that** the pin (19) has a semicircular profile.

5. The dry powder inhaler device (11) according to any of the preceding claims, **characterized in that** the main drum (5) is connected to a transmission wheel (22) disposed on the transmission arm (20).

6. The dry powder inhaler device (11) according to any of the preceding claims, **characterized by** comprising a pretensioned spring which restores the trigger (2) to its initial position after it is pushed in and released by a user.

7. The dry powder inhaler device (11) according to Claim 6, wherein the spring is disposed at a lower side of the transmission wheel (22).

8. The dry powder inhaler device (11) according to any of the preceding claims, **characterized in that** the pin (19) provided on the second end (18) of the transmission arm (20) is moved by sliding on the surface of the triangular-shaped second end (13) of the pivot arm (16).

9. The dry powder inhaler device (11) according to any of the preceding claims, wherein the pivot arm (16) and the transmission arm (20) are disposed between the main drum (5) and the trigger (2).

## Patentansprüche

1. Inhalationseinrichtung (11) für Trockenpulver, mit einem Körper (1) und einem Auslöser (2), der sich in linearer Richtung in dem Körper (1) bewegt, wobei ein innerer Körper (25) im Inneren des äußeren Körpers (1) angeordnet ist, einer Haupttrommel (5), in welcher ein Blisterstreifen (4) angeordnet ist, welcher Medikamente tragende Vertiefungen (3) hat, einem Zahnradsatz (6, 7, 8), der es ermöglicht, Medikamente, die in den Vertiefungen des zu handhabenden Blisterstreifens enthalten sind, durch Drehen der Haupttrommel (5) freizugeben, um welche herum eine Schutzschicht oder Abdeckung des Blisterstreifens (4) gewickelt ist, und mit Zusatzrädern (9, 10), die mit der Haupttrommel (5) in Verbindung stehen, und um welche herum eine Hauptschicht des Blisterstreifens (4) gewickelt ist, **dadurch gekennzeichnet, dass** die Vorrichtung weiterhin aufweist:
- einen Schwenkarm (16), der mit Hilfe eines Trägers/Zapfens (14) zwischen einem ersten Ende (12) und einem zweiten Ende (13) des Schwenkarmes an den Körper (1) angeschlossen ist, wobei der Schwenkarm (16) die lineare Bewegung des Auslösers (2) mit Hilfe eines Zapfens (15), der an dem Auslöser (2) angeordnet ist, in eine Drehbewegung an seinem zweiten Ende (13) umsetzt, und
- einen Übertragungsarm (20), der ein erstes Ende (17) hat, welches mit der Trommel (5) in drehbarer Weise verbunden ist, und ein zweites Ende (18) hat, das einen Zapfen (19) aufweist, der so angeordnet ist, dass er an dem zweiten Ende (13) des Schwenkarmes (16) anliegt, wobei der Übertragungsarm (20) die Drehbewegung, die er von dem Schwenkarm (16) erfährt, auf die Trommel (5) überträgt,
sodass die lineare Bewegung des Auslösers (2), die in eine Drehbewegung umgewandelt wurde, auf den Zahnradsatz (6, 7, 8) übertragen wird.

2. Inhalationsvorrichtung (11) für Trockenpulver nach Anspruch 1, **dadurch gekennzeichnet, dass** der Auslöser (2) eine Barriere (21) aufweist, welche die Drehung des Schwenkarms (16) einschränkt.

3. Inhalationsvorrichtung (11) für Trockenpulver nach einem der vorstehenden Ansprüche, **dadurch gekennzeichnet, dass** das zweite Ende (13) des Schwenkarmes (16) eine dreieckige Form hat.

4. Inhalationsvorrichtung (11) für Trockenpulver nach einem der vorstehenden Ansprüche, **dadurch gekennzeichnet, dass** der Zapfen (19) ein halbkreisförmiges Profil hat.

5. Inhalationsvorrichtung (11) für Trockenpulver nach einem der vorstehenden Ansprüche, **dadurch gekennzeichnet, dass** die Haupttrommel (5) mit einem Übertragungsrad (22) verbunden ist, welches an dem Übertragungsarm (20) angeordnet ist.

6. Inhalationsvorrichtung (11) für Trockenpulver nach einem der vorstehenden Ansprüche, **dadurch gekennzeichnet, dass** sie eine vorgespannte Feder aufweist, welche den Auslöser (2), nachdem er linear gedrückt und durch einen Benutzer freigegeben wurde, in seine Anfangsposition zurückbringt.

7. Inhalationsvorrichtung (11) für Trockenpulver nach Anspruch 6, wobei die Feder an einer unteren Seite des Übertragungsrades (22) angeordnet ist.

8. Inhalationsvorrichtung (11) für Trockenpulver nach einem der vorstehenden Ansprüche, **dadurch gekennzeichnet, dass** der Zapfen (19), der an dem zweiten Ende (18) des Übertragungsarmes (20) vorgesehen ist, auf der Oberfläche des dreieckig geformten zweiten Endes (13) des Schwenkarmes (16) durch Gleiten bewegt wird.

9. Inhalationsvorrichtung (11) für Trockenpulver nach einem der vorstehenden Ansprüche, wobei der Schwenkarm (16) und der Übertragungsarm (20) zwischen der Haupttrommel (5) und dem Auslöser (2) angeordnet sind.

## Revendications

1. Dispositif d'inhalation de poudre sèche (11) comprenant un corps (1) et un élément de déclenchement (2) se déplaçant dans une direction linéaire sur le corps (1), un corps interne (25) positionné à l'intérieur du corps externe (1), un tambour principal (5) dans lequel une bande de capsules de conditionnement (4) comportant des cavités de conditionnement de médicament (3) est placée, un jeu d'engrenages (6, 7, 8) qui permet la libération de médicaments contenus dans les cavités de ladite bande de capsules de conditionnement à administrer en faisant tourner le tambour principal (5) et autour desquels une couche ou couvercle de protection de la bande de capsules de conditionnement (4) est enroulé, et des engrenages supplémentaires (9, 10) qui sont en relation avec le tambour principal (5) et autour desquels une couche principale de la bande de capsules de conditionnement (4) est enroulée, **caractérisé en ce que** ledit dispositif comprend en outre ;
un bras de pivot (16) qui est relié au corps (1) au moyen d'un support/broche (14) entre une première extrémité (12) et une seconde extrémité (13) du bras de pivot (16) de telle sorte que le bras de pivot (16) est amené à tourner autour de ladite broche de support (14), dans lequel ledit bras de pivot (16) transforme le mouvement linéaire à partir de l'élément de déclenchement (2) en un mouvement de rotation sur sa seconde extrémité (13) au moyen d'une broche (15) disposée sur l'élément de déclenchement (2), et
un bras de transmission (20) qui comporte une première extrémité (17) raccordée au tambour (5) d'une manière à pouvoir tourner et une seconde extrémité (18) comprenant une broche (19) disposée de manière à reposer contre la seconde extrémité (13) du bras de pivot (16), dans lequel ledit bras de transmission (20) transmet le mouvement de rotation reçu à partir du bras de pivot (16) au tambour (5)
de telle sorte que le mouvement linéaire de l'élément de déclenchement (2) transformé en un mouvement de rotation est transféré au jeu d'engrenages (6, 7, 8).

2. Dispositif d'inhalation de poudre sèche (11) selon la revendication 1, **caractérisé en ce que** l'élément de déclenchement (2) comprend une barrière (21) qui limite la rotation du bras de pivot (16).

3. Dispositif d'inhalation de poudre sèche (11) selon l'une quelconque des revendications précédentes, **caractérisé en ce que** la seconde extrémité (13) du bras de pivot (16) présente une forme triangulaire.

4. Dispositif d'inhalation de poudre sèche (11) selon l'une quelconque des revendications précédentes, **caractérisé en ce que** la broche (19) présente un profil semi-circulaire.

5. Dispositif d'inhalation de poudre sèche (11) selon l'une quelconque des revendications précédentes, **caractérisé en ce que** le tambour principal (5) est relié à une roue de transmission (22) disposée sur le bras de transmission (20).

6. Dispositif d'inhalation de poudre sèche (11) selon l'une quelconque des revendications précédentes, **caractérisé en ce qu'**il comprend un ressort de précharge qui ramène l'élément de déclenchement (2) à sa position initiale après qu'il a été poussé et libéré par un utilisateur.

7. Dispositif d'inhalation de poudre sèche (11) selon la revendication 6, dans lequel le ressort est disposé sur un côté inférieur de la roue de transmission (22).

8. Dispositif d'inhalation de poudre sèche (11) selon l'une quelconque des revendications précédentes, **caractérisé en ce que** la broche (19) agencée sur la seconde extrémité (18) du bras de transmission (20) est déplacée en coulissant sur la surface de la seconde extrémité de forme triangulaire (13) du bras de pivot (16).

9. Dispositif d'inhalation de poudre sèche (11) selon l'une quelconque des revendications précédentes, dans lequel le bras de pivot (16) et le bras de transmission (20) sont disposés entre le tambour principal (5) et l'élément de déclenchement (2).
